# EUROPÄISCHE PATENTSCHRIFT

(11) **EP 0 742 709 B1**
(45) Veröffentlichungstag und Bekanntmachung des Hinweises auf die Patenterteilung: **22.09.1999**
(21) Anmeldenummer: 95907633.2
(22) Anmeldetag: 27.01.1995
(51) Int. Cl.: A61K 7/06, A61K 7/13

(54) **WIRKSTOFFKOMPLEX FÜR HAARBEHANDLUNGSMITTEL**
COMPLEX OF ACTIVE INGREDIENTS FOR HAIR CONDITIONERS
COMPLEXE DE PRINCIPES ACTIFS POUR AGENTS DE SOINS CAPILLAIRES

(30) Priorität: 05.02.1994 DE 4403570
(43) Veröffentlichungstag der Anmeldung: 20.11.1996
(73) Patentinhaber: Henkel Kommanditgesellschaft auf Aktien, 40191 Düsseldorf (DE)
(72) Erfinder: HÖFFKES, Horst, D-40595 Düsseldorf (DE); SEIDEL, Kurt, D-40589 Düsseldorf (DE); OBERKOBUSCH, Doris, D-40591 Düsseldorf (DE); HOLLENBERG, Detlef, D-40699 Erkrath (DE); PRIEBE, Christian, D-42489 Wülfrath (DE)
(86) Internationale Anmeldenummer: EP9500334
(87) Internationale Veröffentlichungsnummer: WO9520938

(56) Entgegenhaltungen:
- EP-A- 0 188 216
- DE-A- 3 503 618
- FR-A- 2 567 753
- STN, Datenbank Liefferant, Karlsruhe, DE, Datenbank Chemical Abstracts, Band 113, N 154830 Siehe die Zusammenfassung & JP,A,02 084 496 (KAO CORP.)

## Beschreibung

Die Erfindung betrifft die Verwendung einer speziellen Wirkstoffkombination in Haarbehandlungsmitteln.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen.

So ist beispielsweise die biologische Abbaubarkeit der quaternären Ammoniumverbindungen unbefriedigend und ihre mangelnde Kompatibilität mit Aniontensiden schränkt die Formulierungsmöglichkeiten stark ein. Die gleichen Nachteile gelten auch für die kationischen Polymeren. Weiterhin können bei Polymeren unerwünschte Kumulationen auf dem Haar auftreten.

Alkylphosphonate und Alkylphosphate sind zwar mit anionischen Tensiden kompatibel, verfügen aber nur über begrenzte pflegende Eigenschaften.

Es besteht daher weiterhin ein Bedarf nach Wirkstoffen bzw. Wirkstoffkombinationen mit guten pflegenden Eigenschaften und guter biologischer Abbaubarkeit, bei denen unerwünschte Kumulationen auf dem Haar ausgeschlossen sind.

Es wurde nun überraschenderweise gefunden, daß eine Kombination aus bestimmten Dicarbonsäuren und wasserunlöslichen Ölen oder Wachsen diese Anforderungen in hervorragender Weise erfüllt.

Gegenstand der Erfindung ist daher die Verwendung einer Wirkstoffkombination, bestehend aus
(A) einer Dicarbonsäure der allgemeinen Formel (I), 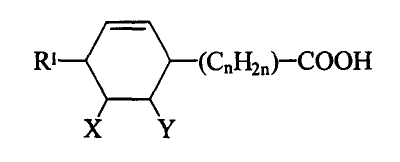 in der R¹ steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, einer Dicarbonsäure der allgemeinen Formel (I), die zusätzlich noch 1 bis 3 Methyl- oder Ethylsubstituenten am Cyclohexenring trägt, einer Dicarbonsäure, die aus den Carbonsäuren gemäß Formel (I) formal durch Anlagerung eines Moleküls Wasser an die Doppelbindung im Cyclohexenring entsteht, oder einem physiologisch verträglichen Salz einer dieser Säuren
   und
(B) einem wasserunlöslichen Öl oder Wachs,
   in Haarbehandlungsmitteln, die, wenn sie frei von Haarfarbstoffen sind, keine wasserlöslichen, ionischen Polymeren enthalten.

Dicarbonsäuren der Formel (I) sind in der Literatur bekannt.

Ein Herstellungsverfahren ist beispielsweise der US-Patentschrift 3,753,968 zu entnehmen. Die deutsche Patentschrift 22 50 055 offenbart die Verwendung dieser Dicarbonsäuren in flüssigen Seifenmassen. Aus der deutschen Offenlegungsschrift 28 33 291 sind deodorierende Mittel bekannt, die Zink- oder Magnesiumsalze dieser Dicarbonsäuren enthalten. Schließlich sind aus der deutschen Offenlegungsschrift 35 03 618 Mittel zum Waschen und Spülen der Haare bekannt, bei denen durch Zusatz dieser Dicarbonsäuren eine merklich verbesserte haarkosmetische Wirkung der im Mittel enthaltenen wasserlöslichen ionischen Polymeren enthalten wird. Die dort offenbarten Haarspülmittel können auch wasserunlösliche, kosmetische Öl- und Fettkomponenten enthalten, deren Solubilisierung durch die Dicarbonsäuren erheblich verbessert wird.

Keiner dieser Druckschriften ist aber der geringste Hinweis auf die überraschenden haarkosmetischen Effekte der erfindungsgemäßen Wirkstoffkombination zu entnehmen.

Die Carbonsäuren der Formel (I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Dabei bietet sich vor allen die aus natürlichen Fetten und Ölen zugängliche Linolsäure an. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Es sind solche Dicarbonsäuren der Formel (I) bevorzugt, bei denen R¹ steht für eine lineare oder methylverzweigte, gesättigte Alkylgruppe mit 4 bis 8 Kohlenstoffatomen und n für eine Zahl von 6 bis 10.

Erfindungsgemäß einsetzbar neben den, bevorzugten, Dicarbonsäuren gemäß Formel (I) sind auch solche Dicarbonsäuren, die sich von den Verbindungen gemäß Formel (I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{R} 1550 und Westvaco Diacid^{R} 1595 (Hersteller: Westvaco) erhältlich.

Neben den Dicarbonsäuren selbst können auch deren physiologisch verträgliche Salze erfindungsgemäß eingesetzt werden. Beispiele für solche Salze sind die Alkali-, Erdalkali-, Ammonium-, die Mono-, Di- und Trimethyl-, -ethyl- und -hydroxyethyl-Ammoniumsalze sowie das Zinksalz. Die Natrium-, Kalium- und Ammoniumsalze sind bevorzugte Salze. Weiterhin kann es aus Formulierungsgründen bevorzugt sein, die Dicarbonsäurekomponente (A) aus den wasserlöslichen Vertretern, insbesondere den wasserlöslichen Salzen auszuwählen. Das Kaliumsalz der 5- und 6-Carboxy-4-hexyl-2-cyclohexen-1-octansäure, das als wäßrige Lösung unter der Bezeichnung Westvaco Diacid^{R} H-240 kommerziell erhältlich ist, hat sich als besonders geeignet erwiesen.

Die zweite Komponente der erfindungsgemäßen Wirkstoffkombination ist ein wasserunlösliches Fett oder Öl. Als wasserunlöslich im Sinne der Anmeldung sind solche Stoffe zu verstehen, deren Löslichkeit in Wasser bei 20 °C 1 g/l oder weniger beträgt. Stoffe mit einer Löslichkeit von 0,1 g/l und weniger sind bevorzugt.

Als wasserunlösliche Fette und Öle kommen beispielsweise natürliche Fette und Öle, Mono-, Di- und Triglyceride von gesättigten und ungesättigten Fettsäuren, Ethylenglykolester von Fettsäuren, Fettsäuren, niedrig ethoxylierte Fettsäuren, Fettalkohole, niedrig ethoxylierte Fettalkohole, Cholesterin und niedrig ethoxylierte Cholesterine, aber auch synthetische Produkte wie beispielsweise Silikonöle in Betracht. Als besonders wirksam haben sich Fettsäuren, niedrig ethoxylierte Fettsäuren, Fettalkohole und niedrig ethoxylierte Fettalkohole erwiesen. Als Komponente (B) können auch entsprechende Mischungen verwendet werden.

Beispiele für Komponenten (B) sind Laurylalkohol, Myristylalkohol, Cetylalkohol, Stearylalkohol, Mischungen von Cetyl/Stearylalkohol, Talgfettalkohol, Kokosfettalkohol, Palmitinsäure, Stearinsäure, Jojobaöl, Paraffinöl und Polydimethylsiloxane.

Langkettige Fettalkohole wie Cetylalkohol und Stearylalkohol, deren Mischungen sowie natürliche Fettalkoholmischungen mit einem hohen Gehalt an diesen Komponenten sind bevorzugte Komponenten (B).

Die Dicarbonsäuren (A) sind in den erfindungsgemäß verwendeten Mitteln üblicherweise in Mengen von 0,1 bis 10 Gew.-%, bezogen auf das Mittel, enthalten. In vielen Fällen haben sich Mengen von 1 bis 5 Gew.-% als besonders vorteilhaft erwiesen.

Die wasserunlöslichen Fette und Öle (B) sind in den erfindungsgemäß verwendeten Mitteln bevorzugt in Mengen von 0,1 bis 15 Gew.-%, bezogen auf das Mittel, enthalten. Mengen von 3 bis 10 Gew.-% sind besonders bevorzugt.

Neben den obligatorischen Komponenten (A) und (B) können die erfindungsgemäß verwendeten Haarbehandlungsmittel alle für solche Mittel üblichen Komponenten, mit Ausnahme von wasserlöslichen ionischen Polymeren, enthalten. Die Auswahl dieser weiteren Komponenten wird im wesentlichen durch die Art des Haarbehandlungsmittels bestimmt.

Es hat sich jedoch gezeigt, daß praktisch unabhängig von der Art des Haarbehandlungsmittels bestimmte weitere Komponenten die erfindungsgemäße Wirkstoffkombination in ihrer Wirkung besonders vorteilhaft ergänzen.

Eine Klasse von solchen Komponenten stellen die nichtionogenen Tenside dar.

Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und oligoglycoside und deren ethoxylierte Analoga sowie
- Anlagerungsprodukte von 5 bis 60 Mol Ethylenoxid an Rizinusöl und gehärtetes Rizinusöl.

Bei diesen Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den nichtionogenen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Alkylpolyglykoside gemäß der Formel RO-(Z)ₓ, in der R steht für einen Alkylrest mit 8 bis 22 Kohlenstoffatomen, Z für einen Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Motto- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Ebenfalls als besonders vorteilhaft haben sich Haarbehandlungsmittel erwiesen, die neben der erfindungsgemäßen Wirkstoffkombination Acyllactylate der allgemeinen Formel 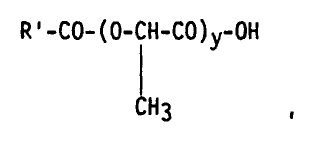 in der R' für eine lineare oder verzweigte, gesättigte oder ungesättigte Alkyl- oder Alkenylgruppe mit 6 bis 22 Kohlenstoffatomen steht und y für eine Zahl vonb 1 bis 5, enthalten.

Acyllactylate, bei denen R für eine, insbesondere gesättigte, lineare oder methylverzweigte Alkylgruppe mit 12 bis 18 Kohlenstoffatomen steht und y für eine Zahl von 1 bis 3, sind besonders bevorzugt.

Solche Acyllactylate sind im Handel unter dem Warenzeichen Pationic^{R} erhältlich.

Ein Natrium-isostearoyllactyllactylat, das unter der Bezeichnung Pationic^{R}ISL vertrieben wird, ist ein besonders bevorzugtes Acyllactylat.

Schließlich haben sich auch erfindungsgemäße Mittel, die zusätzlich nichtionogene Polymere vom Typ Stärke bzw. Cyclodextrin enthalten, als besonders wirksam erwiesen.

Die Art des erfindungsgemäß verwendeten Haarbehandlungsmittels unterliegt keinen Beschränkungen. Die erfindungsgemäßen Mittel können sowohl auf dem Haar verbleiben, als auch nach einer Einwirkzeit von wenigen Sekunden bis Minuten wieder ausgespült werden. Beispiele für erfindungsgemäß verwendete Mittel sind Shampoos, Spülungen, Kuren, Konditioniermittel, Tönungsmittel, Färbemittel, Dauerwellmittel, Fixiermittel, Haarsprays und Fönwellen.

Weitere Komponenten dieser Mittel können dann beispielsweise sein:
- anionische Tenside, wie beispielsweise Fettalkylsulfate und -ethersulfate sowie Alkylethercarbonsäuren,
- kationische Tenside, wie beispielsweise quartäre Ammoniumverbindungen, Amidoamine und quaternisierte Ester und Proteinhydrolysate,
- zwitterionische Tenside, wie beispielsweise Betaine,
- ampholytische Tenside,
- nichtionische Polymere wie beispielsweise Polyvinylpyrrolidon, Vinylpyrrolidon/Vinylacetat-Copolymere und Celluloseether,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Die erfindungsgemäß verwendeten Mittel können beispielsweise als wäßrige, alkoholische oder wäßrig-alkoholische Lösungen, Cremes, Gele oder Emulsionen formuliert sein.

Als besonders vorteilhaft hat sich die erfindungsgemäße Wirkstoffkombination in Mitteln zum Färben und Tönen von Haaren erwiesen.

Solche Mittel enthalten entweder sogenannte "direktziehende" Farbstoffe und/oder Vorprodukte für Oxidationsfarbstoffe.

Übliche direktziehende Farbstoffe sind z. B. Nitrobenzolderivate, Andrachinon-Farbstoffe, Triphenylmethanfarbstoffe und Azofarbstoffe. In Haarfärbe- und Tönungsmitteln eingesetzte Vertreter dieser Farbstoffklasse sind beispielsweise die unter den internationalen Bezeichnungen bzw. Handelsnamen HC Yellow 2, HC Yellow 4, Methylgelb, Fluorgelb, Basic Yellow 57, Disperse Orange 3, HC Red 3, HC Red BN, Kardinalrot, Rot Y, Rot X, Basic Red 76, HC Blue 2, Nitroblau, Disperse Blue 3, Basis Blue 99, HC Violet 1, Disperse Violet 1, Disperse Violet 4, Disperse Black 9, Basis Brown 16, Pikraminsäure und Rodol 9 R, bekannten Verbindungen. Weitere geeignete direktziehende Farbstoffe sind beispielsweise die Verbindungen 3-Nitro-4-ethylaminobenzoesäure, 1,2,3,4-Tetrahydro-6-nitro-chinoxalin, 2-Nitro-4-amino-4'-dimethylaminodiphenylamin-2'-carbonsäure und 2-Nitro-4-aminodiphenylamin-2'-carbonsäure.

Als Vorprodukte für Oxidationsfarbstoffe enthalten Haarfärbemittel sogenannte Entwickler- und Kupplerkomponenten. Die Etttwicklerkomponenten bilden unter dem Einfluß von Oxidatiottsmitteln oder Luftsauerstoff untereinander oder unter Kupplung mit einer oder mehreren Kupplerkomponenten die eigentlichen Farbstoffe aus.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl3-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol und 2-Methylresorcin.

Die folgenden Beispiele sollen den Gegenstand der Erfindung näher erläutern.

### Beispiele

### 1. Untersuchungsverfahren

### 1.1 Gezielte Schädigung von Haarsträhnen

Trockene Haarsträhnen von ca. 2 g Gewicht (Fischbach und Miller Typ 6923) wurden einmal mit 32 g Blondiermischung (Marktprodukt Poly Blond Medium Aufheller) eine halbe Stunde lang blondiert. Nach dem Auswaschen der Blondiermischung wurden die Haarsträhnen unmittelbar ohne Zwischentrocknung einer Dauerwellbehandlung mit dem Marktprodukt Poly Lock Normal unterzogen. Die Einwirkdauern von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

### 1.2 Prüfung der Naßkämmbarkeit

Die gespülte Haarsträhne wurde vor der Prüfung mit je 0,2 ml einer 50%igen Lösung von Texapon^{R} N25 (28%ige Lösung von Natriumlaurylethersulfat in Wasser)-Lösung intensiv shampooniert und anschließend ausgespült. Danach wurden 0,5 ml der zu prüfenden Formulierung gleichmäßig in das Haar massiert, eine Minute im Haar belassen und dann sorgfältig ausgespült. Danach wurde mit einem feinzinkigen Kamm gekämmt und der Kämmwiderstand subjektiv beurteilt. Anschließend wurde an derselben Strähne in gleicher Weise eine Standardformulierung als Vergleich geprüft. Die Beurteilung wurde entsprechend einem Beurteilungssystem von 1 (= sehr gut) bis 6 (= ungenügend) abgegeben.

### 2. Formulierungen und Ergebnisse

Die Mengenangaben in den folgenden Formulierungen sind jeweils Gew.-%.

### 2.1 Haarspülung

| | |
|---|---|
| Westvaco Diacid^{R} H-240¹ | 10,0 |
| Jojobaöl | 0,5 |
| Stenol^{R} 1618² | 6,0 |
| Parfümöl | 0,15 |
| Euxyl^{R}K400³ | 0,1 |
| Wasser | ad 100 |

| | |
|---|---|
| ¹ Kaliumsalz der 4-Hexyl-5(6)-carboxy-2-cyclohexen-1-octansäure (ca. 41 % Aktivsubstanz in Wasser) (WESTVACO) | |
| ² C₁₆/C₁₈-Fettalkohol im Verhältnis 1:1 (HENKEL) | |
| ³ 1,2-Dibrom-2,4-dicyanobutan (SCHÜLKE & MAYR) | |

Die Naßkämmbarkeit der mit dieser Formulierung behandelten Haare wurde mit sehr gut bis gut benotet.

### 2.2 Haarspülung

| | |
|---|---|
| Westvaco Diacid^{R} H-240 | 10,0 |
| Stenol^{R} 1618 | 3,0 |
| Pationic^{R} ISL 4 | 5,0 |
| Parfümöl | 0,15 |
| Euxyl^{R}K400 | 0,1 |
| Wasser | ad 100 |

| | |
|---|---|
| ⁴ Natriumsalz des Isostearoyllactyllactylates (CTFA-Bezeichnung: Sodium Isostearoyl-2-lactate) (PATCO) | |

Die Naßkämmbarkeit der mit dieser Spülung behandelten Haare wurde als sehr gut (Note 1) bewertet. Der Frisurenhalt war deutlich verbessert.

### 2.3 Haarspülung

| | |
|---|---|
| Diacid H-240 | 10,0 |
| Stenol 1618 | 3,0 |
| Jojobaöl | 0,5 |
| Lösliche Stärke | 3,0 |
| Parfümöl | 0,15 |
| Konservierungsmittel | 0,1 |
| Wasser | ad 100 |

Die Naßkämmbarkeit der mit dieser Spülung behandelten Haare wurde als sehr gut (Note 1) bewertet. Der Frisurenhalt war deutlich verbessert.

### 2.4 Haarkur

| | |
|---|---|
| Westvaco Diacid^{R} H-240 | 10,0 |
| Stenol^{R} 1618 | 5,0 |
| Silikonöl^{R} DC 200⁵ | 2,0 |
| Plantaren 600^{R} 6 | 2,0 |
| 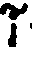 -Cyclodextrin | 3,0 |
| Natrosol^{R} 250 HR⁷ | 0,5 |
| Parfümöl | q.s |
| Wasser | ad 100 |

| | |
|---|---|
| ⁵ Hexamethyl-Disiloxan (CTFA-Bezeichnung: Hexamethyl-Bisiloxane) (DOW CORNING) | |
| ⁶ C₁₂₋₁₆-Fettalkohol-1,4-glucosid (CTFA-Bezeichnung: Lauryl Polyglycose; ca. 52 % Aktivsubstanz in Wasser) (HENKEL) | |
| ⁷ Hydroxyethylcellulose (HENKEL) | |

Die Naßkämmbarkeit der mit dieser Haarkur behandelten Haare war sehr gut (Note 1); die Haarkur verlieh dem Haar zusätzlich eine leichte Festigung.

### 2.5 Haarshampoo

| | |
|---|---|
| Westvaco Diacid^{R} H-240 | 10,0 |
| Texapon^{R} N 25⁸ | 30,0 |
| Plantaren^{R} 600 | 3,0 |
| Akypo^{R} Soft 45 NV⁹ | 7,0 |
| Pationic^{R} ISL | 3,0 |
| Stenol 1618 | 3,0 |
| Parfümöl | q.s. |
| Konservierungsmittel | q.s. |
| Wasser | ad 100 |

| | |
|---|---|
| ⁸ Natriumlaurylethersulfat (ca. 28 % Aktivsubstanz; CTFA-Bezeichnung: Sodium Laureth Sulfate) (HENKEL) | |
| ⁹ C₁₂₋₁₄-Fettalkohol-4,5-Ethylenoxid-essigsäure-Natriumsalz (CTFA-Bezeichnung: Sodium Laureth-6 Carboxylate; 21 % Aktivsubstanz in Wasser) (CHEM-Y) | |

Die Naßkämmbarkeit der mit diesem Shampoo behandelten Haare wurde mit befriedigend (Note 3) bewertet. Eine Behandlung mit diesem Shampoo führte außerdem zu einem verbesserten Halt der Frisur.

### 2.6 Haarshampoo

| | |
|---|---|
| Westvaco Diacid^{R} H-240 | 10,0 |
| Texapon^{R} N 25 | 40,0 |
| Stenol 1618 | 1,0 |
| Natriumstearat | 3,0 |
| Behensäure | 1,5 |
| Stärke, löslich | 1,0 |
| Parfümöl | q.s. |
| Konservierungsmittel | q.s. |
| Wasser | ad 100 |

Die Naßkämmbarkeit der mit diesem Shampoo behandelten Haare wurde mit befriedigend (Note 3) bewertet. Eine Behandlung mit diesem Shampoo führte außerdem zu einem verbesserten Halt der Frisur.

### 2.7 Haartönungsmittel

| | Erfindung | Vergleich |
|---|---|---|
| Texapon N 25 | - | 16,0 |
| Diacid H-240 | 10 | - |
| Stenol 1618 | 5,0 | 6,0 |
| Stärke, löslich | 3,0 | 3,0 |
| 4-Amino-2-nitrodiphenyl- | | |
| amin-2'-carbonsäure | 0,1 | 0,1 |
| 6-Nitro-1,2,3,4-tetrahydrochinoxalinhydrochlorid | 0,1 | 0,1 |
| Wasser | ad 100 | ad 100 |
| mit Ammoniak auf | pH 7,51 | pH 7,53 |

Beide Rezepturen wurden mit 4 % Treibgas (Propan-Butan) als Aerosole formuliert.

Obwohl beide Rezepturen den gleichen Aktivsubstanzgehalt an Tensid enthielten, wurden die Naßkämmbarkeiten von Haaren, die mit der erfindungsgemäßen Rezeptur behandelt wurden, mit gut bis befriedigend (Note 2 bis 3) bewertet, während die Naßkämmbarkeit nach Behandlung mit der Vergleichsrezeptur nur mit ausreichend (Note 4) bewertet wurde. Weiterhin wies die erfindungsgemäße Rezeptur bei Ausfärbung auf lichtblondem Haar (Alcinko, Virgin White) eine größere Farbtiefe auf (L* nach Cielab = 47,312 für den Vergleich und 46,453 für die erfindungsgemäße Formulierung).

### 2.8 Haarfärbemittel

| | |
|---|---|
| Westvaco Diacid^{R} H-240 | 10,0 |
| Plantaren^{R} 600 | 2,0 |
| Hydrenol^{R} D¹⁰ | 5,0 |
| p-Toluylendiaminsulfat | 0,1 |
| p-Aminophenolhydrochlorid | 0,03 |
| Resorcin | 0,04 |
| m-Aminophenol | 0,004 |
| Stabilisierungsmittel, Parfümöl, Puffersalz | q.s. |
| Wasser | ad 100 |
| Mit Ammoniak wurde ein pH-Wert von 9,5 eingestellt. | |

| | |
|---|---|
| ¹⁰ C₁₆/C₁₈-Fettalkohol im Verhältnis 1:2 (HENKEL) | |

Das Haarfärbemittel wurde mit einer kommerziellen 6%igen Wasserstoffperoxiddispersion (Poly Color Brillance Color-Creme, Mischungsverhältnis 1:1) ausgefärbt. Es resulierte eine hellblonde Nuance. Die Naßkämmbarkeit des mit diesem Färbemittel behandelten Haares wurde mit gut bis befriedigend (Note 2 bis 3) bewertet.

### 2.9 Wellcreme

| | |
|---|---|
| Diacid H-240 | 2,0 |
| Kokosfettalkohol | 1,0 |
| Thioglycolsäure | 8,0 |
| Eumulgin^{R} B 2¹¹ | 1,0 |
| Dehyton^{R} K¹² | 3,0 |
| Natrosol-250 HR | 1,5 |
| Harnstoff | 3,0 |
| EDTA | 0,3 |
| Parfümöl | 0,3 |
| Wasser | ad 100 |
| Mit einer Ammoniaklösung wurde ein pH-Wert von 8,5 eingestellt. | |

| | |
|---|---|
| ¹¹ Cetylstearylalkohol mit ca. 20 Mol E0 (CTFA-Bezeichnung: Ceteareth-20) (HENKEL) | |
| ¹² Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | |

Das mit dieser Wellcreme behandelte, nicht vorgeschädigte Haar wies auch nach der Fixierung mit einer kommerziellen Fixierlösung einen leichten Pflegeeffekt auf.

### 2.10 Fixierung

| | |
|---|---|
| Diacid H-240 | 4,0 |
| Stenol 1618 | 2,0 |
| Texapon N 25 | 2,0 |
| Wasserstoffperoxid (50%ig) | 5,0 |
| Stabilisator | q.s. |
| Parfümöl | q.s. |
| Wasser | ad 100 |

Der pH-Wert wurde mit Phosphorsäure auf 5 eingestellt.

Auch hier wurden die Eigenschaften an nicht vorgeschädigtem Haar geprüft. Sowohl Kämmbarkeit als auch Griff sind gegenüber einer Vergleichsformulie rung ohne Westvaco Diacid H-210 deutlich verbessert.

## Patentansprüche

1. Verwendung einer Wirkstoffkombination, bestehend aus
(A) einer Dicarbonsäure der allgemeinen Formel (I), 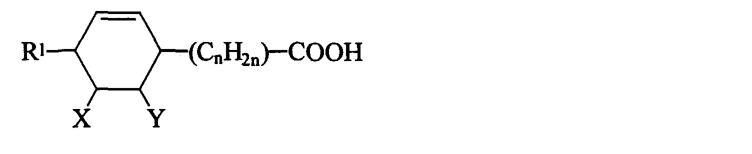 in der R¹ steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, einer Dicarbonsäure der allgemeinen Formel (I), die zusätzlich noch 1 bis 3 Methyl- oder Ethylsubstituenten am Cyclohexenring trägt, einer Dicarbonsäure, die aus den Carbonsäuren gemäß Formel (I) formal durch Anlagerung eines Moleküls Wasser an die Doppelbindung im Cyclohexenring entsteht, oder einem physiologisch verträglichen Salz einer dieser Säuren
und
(B) einem wasserunlöslichen Öl oder Wachs,
in Haarbehandlungsmitteln, die, wem sie frei von Haarfarbstoffen sind, keine wasserlöslichen, ionischen Polymeren sind enthalten.

2. Verwendung nach Anspruch 1, dadurch gekennzeichnet, daß R¹ steht für eine lineare oder methylverzweigte, gesättigte Alkylgruppen mit 4 bis 8 Kohlenstoffatomen und n für eine Zahl von 6 bis 10.

3. Verwendung nach Anspruch 1, dadurch gekennzeichnet, daß die Dicarbonsäure gemäß Formel (I) ein Reaktionsprodukt ist, daß durch Diels-Alder-Reaktion aus Littolsäure und Acrylsäure erhältlich ist.

4. Verwendung nach einem der Ansprüche 1 bis 3, dadurch gekennzeichnet, daß das physiologisch verträgliche Salz der Dicarbonsäure gemäß Formel (I) ein Alkali- oder Ammoniumsalz ist.

5. Verwendung nach einem der Ansprüche 1 bis 4, dadurch gekennzeichnet, daß die Komponente (B) ausgewählt ist aus der Gruppe, die von Fettalkoholen, Fettsäuren, ethoxylierten Fettalkoholen und ethoxylierten Fettsäuren.

6. Verwendung nach einem der Ansprüche 1 bis 5, dadurch gekennzeichnet, daß die Komponente (A) in Mengen von 0,1 bis 10 Gew.-%, insbesondere von 1 bis 5 Gew.-%, jeweils bezogen auf das gesamte Haarbehandlungsmittel, enthalten ist.

7. Verwendung nach einem der Ansprüche 1 bis 6, dadurch gekennzeichnet, daß die Komponente (B) in Mengen von 0,1 bis 15 Gew.-%, insbesondere von 3 bis 10 Gew.-%, jeweils bezogen auf das gesamte Haarbehandlungsmittel, enthalten ist.

8. Verwendung nach einem der Ansprüche 1 bis 7, dadurch gekennzeichnet, daß das Haarbehandlungsmittel weiterhin ein nichtionogenes Tensid, insbesondere ein Alkylpolyglykosid, enthält.

9. Verwendung nach einem der Ansprüche 1 bis 8, dadurch gekennzeichnet, daß das Haarbehandlungsmittel weiterhin ein Acyllactylat der allgemeinen Formel 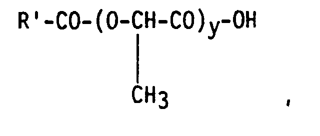 in der R' für eine lineare oder verzweigte, gesättigte oder ungesättigte Alkyl- oder Alkenylgruppe mit 6 bis 22 Kohlenstoffatomen steht und y für eine Zahl von 1 bis 5, enthält.

10. Verwendung nach einem der Ansprüche 1 bis 9, dadurch gekennzeichnet, daß das Haarbehandlungsmittel ein Haarfärbemittel oder ein Haartönungsmittel ist.

## Claims

1. The use of an active-substance combination consisting of
(A) a dicarboxylic acid corresponding to general formula (I): 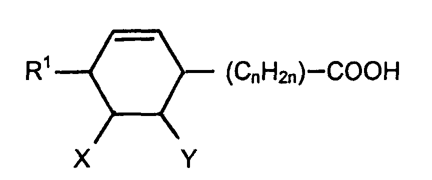 in which R¹ is a linear or branched alkyl or alkenyl group containing 4 to 12 carbon atoms, n is a number of 4 to 12 and one of the two groups X and Y is a COOH group and the other is a hydrogen atom or a methyl or ethyl group,
a dicarboxylic acid corresponding to general formula (I) which additionally contains 1 to 3 methyl or ethyl substituents on the cyclohexene ring, a dicarboxylic acid formally formed from the carboxylic acids corresponding to formula (I) by addition of one molecule of water onto the double bond in the cyclohexene ring or a physiologically compatible salt of one of these acids and
(B) a water-insoluble oil or wax,
in hair treatment compositions which, when free from hair dyes, do not contain any water-soluble ionic polymers.

2. The use claimed in claim 1, characterized in that R¹ is a linear or methyl-branched saturated alkyl group containing 4 to 8 carbon atoms and n is a number of 6 to 10.

3. The use claimed in claim 1, characterized in that the dicarboxylic acid corresponding to formula (I) is a reaction product obtainable by Diels-Alder reaction from linoleic acid and acrylic acid.

4. The use claimed in any of claims 1 to 3, characterized in that the physiologically compatible salt of the dicarboxylic acid corresponding to formula (I) is an alkali metal or ammonium salt.

5. The use claimed in any of claims 1 to 4, characterized in that component (B) is selected from the group consisting of fatty alcohols, fatty acids, ethoxylated fatty alcohols and ethoxylated fatty acids.

6. The use claimed in any of claims 1 to 5, characterized in that component (A) is present in quantities of 0.1 to 10% by weight and more particularly in quantities of 1 to 5% by weight, based on the hair treatment composition as a whole.

7. The use claimed in any of claims 1 to 6, characterized in that component (B) is present in quantities of 0.1 to 15% by weight and more particularly in quantities of 3 to 10% by weight, based on the hair treatment composition as a whole.

8. The use claimed in any of claims 1 to 7, characterized in that the hair treatment composition additionally contains a nonionic surfactant, more especially an alkyl polyglycoside.

9. The use claimed in any of claims 1 to 8, characterized in that the hair treatment composition additionally contains an acyl lactylate corresponding to the following general formula: 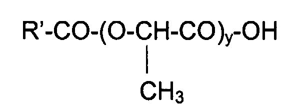 in which R' is a linear or branched, saturated or unsaturated alkyl or alkenyl group containing 6 to 22 carbon atoms and y is a number of 1 to 5.

10. The use claimed in any of claims 1 to 9, characterized in that the hair treatment composition is a hair colorant or a hair tint.

## Revendications

1. Utilisation d'une combinaison de principe actifs formée de :
A) un acide dicarboxylique de formule générale (I) 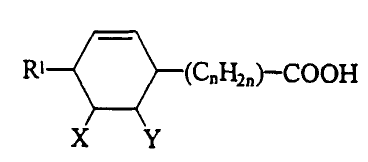 dans laquelle
R¹ représente un groupe alkyle ou alkényle, linéaire ou ramifié, ayant de 4 à 12 atomes de carbone, n représente un nombre allant de 4 à 12 ainsi qu'un des deux groupes X et Y représente un groupe -COOH et l'autre un hydrogène ou un radical méthyle ou éthyle, un acide dicarboxylique de formule générale (I) qui porte en supplément encore 1 à 3 substituants méthyle ou éthyle sur le cycle cyclohexène, un acide dicarboxylique qui se forme à partir des acides carboxyliques conformément à la formule (I) formellement par addition d'une molécule d'eau sur la double liaison dans le cycle cyclohexène,
ou d'un sel physiologiquement compatible d'un de ces acides et,
B) une huile ou une cire insoluble dans l'eau,
dans des agents de traitement capillaire qui, lorsqu'ils sont dépourvus de teintures pour les cheveux, ne renferment aucun polymère ionique soluble dans l'eau.

2. Utilisation selon la revendication 1,
caractérisée en ce que
R¹ représente un radical alkyle saturé, linéaire ou ramifié par un méthyle, ayant de 4 à 8 atomes de carbone et n représente un nombre allant de 6 à 10.

3. Utilisation selon la revendication 1,
caractérisée en ce que
l'acide dicarboxylique conformément à la formule (I) est un produit de réaction qui est accessible par réaction de Diels-Alder, à partir de l'acide linoléique et de l'acide acrylique.

4. Utilisation selon l'une des revendications 1 à 3,
caractérisée en ce que
le sel physiologiquement compatible de l'acide dicarboxylique conformément à la formule (I) est un sel de métal alcalin ou d'ammonium.

5. Utilisation selon l'une des revendications 1 à 4,
caractérisée en ce que
le composant (B) est choisi dans le groupe qui est formé des alcools gras, des acides gras, des alcools gras éthoxylés et des acides gras éthoxylés.

6. Utilisation selon l'une des revendications 1 à 5,
caractérisée en ce que
le composant (A) est contenu en quantités allant de 0,1 à 10 % en poids, en particulier de 1 à 5 % en poids, à chaque fois rapporté à la totalité de l'agent de traitement capillaire.

7. Utilisation selon l'une des revendications 1 à 6,
caractérisée en ce que
le composant (B) est contenu en quantités allant de 0,1 à 15 % en poids, en particulier de 3 à 10 % en poids, à chaque fois rapporté à la totalité de l'agent de traitement capillaire.

8. Utilisation selon l'une des revendications 1 à 7,
caractérisée en ce que
l'agent de traitement capillaire renferme en outre un agent tensioactif non ionogène, en particulier un alkylpolyglycoside.

9. Utilisation selon l'une des revendications 1 à 8,
caractérisée en ce que
l'agent de traitement capillaire renferme en outre un acyllactylate de formule générale, 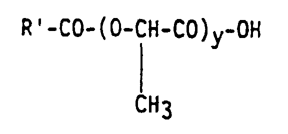 dans laquelle R¹ représente un groupe alkyle ou alkényle linéaire ou ramifié, saturé ou non saturé ayant de 6 à 22 atomes de carbone et y représente un nombre allant de 1 à 5.

10. Utilisation selon l'une des revendications 1 à 9,
caractérisée en ce que
l'agent de traitement capillaire est une teinture pour les cheveux ou un agent de nuançage pour les cheveux.
